# EUROPÄISCHE PATENTSCHRIFT

(11) **EP 1 340 483 B1**
(45) Veröffentlichungstag und Bekanntmachung des Hinweises auf die Patenterteilung: **08.09.2010**
(21) Anmeldenummer: 03001325.4
(22) Anmeldetag: 23.01.2003
(51) Int. Cl.: A61K 6/09, C07D 273/04

(54) **Dentalmaterialien auf der Basis von (Meth)acrylatsubstuierten Iminooxadiazindion-Derivaten**
Dental materials based on (meth)acrylate-substituted iminooxadiazinedione derivatives
Matériaux dentaires à base de dérivés d'iminooxadiazindione substitués avec un groupement (méth)acrylate

(30) Priorität: 27.02.2002 DE 10208395
(43) Veröffentlichungstag der Anmeldung: 03.09.2003
(73) Patentinhaber: Ivoclar Vivadent AG, 9494 Schaan (LI)
(72) Erfinder: Moszner, Norbert, Prof. Dr., 9493 Mauren (LI); Fischer, Urs Karl, 9320 Arbon (CH); Völkel, Thomas, Dr., 88131 Oberreitnau (DE); Rheinberger, Volker, Dr., 9490 Vaduz (LI)
(74) Vertreter: UEXKÜLL & STOLBERG

(56) Entgegenhaltungen:
- EP-A- 0 209 365
- EP-A- 0 262 488
- EP-A- 0 798 299
- EP-A- 1 002 818

## Beschreibung

Die vorliegende Erfindung betrifft Dentalmaterialien auf der Basis von Iminooxadiazindion-Derivaten, die mit (Meth) acrylatresten substituiert sind und die als Vernetzer bei der radikalischen Polymerisation wirksam sind.

Urethan(meth)acrylate finden als Bestandteil von Klebstoffen, Beschichtungen und Dentalmaterialien breite Anwendung (vgl. u.a. R. Holman (Hrsg.), UV and EB Curing Formulation for Printing Inks, Coatings and Paints, SITA-Technology, London 1984, 27; J.P. Fouassier, J.F. Rabek (Hrsg.), Radiation Curing in Polymer Science and Technology, Vol. IV, Elsevier Applied Science, London und New York 1993, 387). Im Dentalbereich wird meist das Monomer UDMA eingesetzt, das durch Umsetzung von 1 mol 2,2,4-Trimethylhexamethylen-diisocyanat mit 2 mol 2-Hydroxyethylmethacrylat (HEMA) zugänglich ist. UDMA wird oft in Kombination mit Bisphenol-A-Diglycidylmethacrylat (Bis-GMA) verwendet, dem Additionsprodukt von Methacrylsäure und Bisphenol-A-diglycidylether (vgl. u.a. DE-A-24 11 760). Grund hierfür sind vor allem die mit Bis-GMA erreichbaren guten mechanischen Eigenschaften.

Die EP 0 266 589 A1 und die EP 0 273 245 A1 offenbaren (Meth)Acrylsäure-Derivate von cycloaliphatischen bzw. aliphatischen Triisocyanaten, die beispielsweise durch Umsetzung von 3 mol HEMA mit 1 mol Triisocyanat zugänglich sind. Die erhaltenen Tri- oder Hexamethacrylaturethanderivate sollen Dentalkomposite mit hoher Abrasionbeständigkeit ergeben.

Weiterhin wurde die Umsetzung von 1 mol Triisocyanat-Isocyanurat, z.B. des Trimeren von Hexamethylendiisocyanat (HDI), mit 1 mol HEMA und 2 mol GDMA zu einem Monomer mit fünf polymerisationsfähigen Gruppen berichtet (S. B. Mitra (ACS, Div. Polym. Chem., Poly. Prepr. 38 (2) (1997), 103). Cyanursäureund Isocyanursäurederivate werden auch als symmetrische Diisocyanat-Trimere bezeichnet.

Aus der DE 197 34 048 A1 und EP 0 798 299 A1 sind asymmetrische Isocyanat-Trimere und Verfahren zu deren Herstellung bekannt. Die asymmetrischen Trimere weisen eine geringe Viskosität auf und sollen sich daher besonders zur Herstellung von Lacken mit einem verringerten Lösemittelgehalt eignen. Außerdem sollen die Verbindungen eine geringe Empfindlichkeit gegenüber Luftfeuchtigkeit zeigen (siehe auch F. Richter, H. Mertes, Farbe&Lacke, 106 (2000) 60).

Der Erfindung liegt die Aufgabe zu Grunde, Dentalwerkstoffe auf der Basis von radikalisch polymerisationsfähigen Monomere zur Verfügung zu stellen, die Polymerisationsprodukte mit guten mechanischen Eigenschaften ergeben .

Erfindungsgemäß wird diese Aufgabe durch Dentalmaterialien gemäß Anspruch 1 auf der Basis von Iminooxadiazindion-Derivaten mit der Formel (I) gelöst, 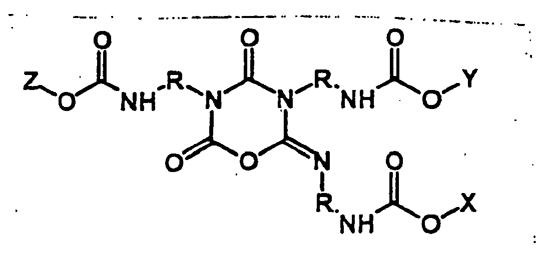 in der
X, Y und Z einen (Meth)acrylsäurerest der Formel (II) 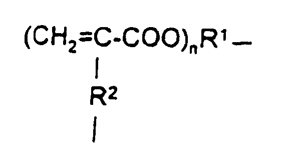 bedeuten, in der
R¹ Wasserstoff, ein aliphatischer Kohlenwasserstoffrest mit 1 bis 14 Kohlenstoffatomen, vorzugsweise 2 bis 5 Kohlenstoffatomen, der durch Sauerstoffatome unterbrochen sein kann, oder ein aromatischer Kohlenwasserstoffrest mit 6 bis 16 Kohlenstoffatomen, vorzugsweise 6 Kohlenstoffatomen ist;
R² Wasserstoff, ein aliphatischer Kohlenwasserstoffrest mit 1 bis 5 Kohlenstoffatomen, vorzugsweise 1 Kohlenstoffatom, oder ein aromatischer Kohlenwasserstoffrest mit 6 Kohlenstoffatomen ist, wobei nur einer der Reste R¹ und R² die Bedeutung Wasserstoff haben kann;
n 1 oder 2 ist;
wobei der (Meth)acrylsäurerest der Formel (II) über R¹ oder R² an den Rest der Formel (I) gebunden und die an R² oder R¹ verbleibende Bindungsstelle durch Wasserstoff abgesättigt ist und wobei X, Y und Z gleich oder verschieden sein können;
die einzelnen Reste R gleich oder verschieden sein können und ausgewählt sind aus alicyclischen oder aromatischen Kohlenwasserstoffresten mit 5 bis 10, vorzugsweise 7 bis 10 Kohlenstoffatomen, oder aliphatischen Kohlenwasserstoffresten mit 1 bis 10 Kohlenstoffatomen, vorzugsweise 6 bis 9 Kohlenstoffatomen.

Der Rest der Formel (II) wird hier der Einfachheit halber auch dann als (Meth)acrylsäurerest bezeichnet wenn R² weder H noch CH₃ ist, obwohl es sich in diesem Fall genau genommen um in α-Position substituierte Acrylat-Reste handelt.

Erfindungsgemäß werden unter aliphatischen Kohlenwasserstoffresten solche Gruppen verstanden, die sich von verzweigten oder unverzweigten Alkanen durch das Entfernen von einem bis drei Wasserstoffatomen ableiten.

Alicyclische Kohlenwasserstoffreste leiten sich entsprechend von den Cycloalkanen ab, vorzugsweise von monocyclischen Cycloalkanen, wobei erfindungsgemäß auch solche Cycloalkane umfaßt werden, die mit Alkylgruppen substituiert sind. Bei alkylsubstituierten Cycloalkanen können das oder die Wasserstoffatome von ringständigen Kohlenstoffatomen oder von Kohlenstoffatomen des oder der Substituenten entfernt werden, d.h. eine Verknüpfung kann über ringständige Kohlenstoffatome oder über Kohlenstoffatome der Seitenkette erfolgen. Bevorzugte alicyclische Kohlenwasserstoffreste sind substituierte oder unsubstituierte Cyclohexanreste. Bevorzugter Substituent ist Methyl.

Unter aromatischen Kohlenwasserstoffresten werden Benzolreste und Reste, die mehrere kondensierte Benzolringe enthalten, verstanden. Die Benzolringe können mit Alkylgruppen substituiert sein (Alkylarylreste). Bei alkylsubstituierten aromatischen Resten können das oder die Wasserstoffatome von ringständigen Kohlenstoffatomen, wie z.B. bei dem MethylphenylRest, oder von Kohlenstoffatomen des oder der Substituenten entfernt werden, wie z.B. bei dem Benzyl-Rest, d.h. eine Verknüpfung kann über ringständige Kohlenstoffatome oder über Kohlenstoffatome der Seitenkette erfolgen. Bevorzugte aromatische Kohlenwasserstoffreste sind substituierte oder unsubstituierte Benzolreste. Bevorzugter Substituent ist Methyl.

Der (Meth)acrylsäurerest der Formel (II) weist vorzugsweise eine Struktur der Formel (IIa), (IIb) oder (IIc) auf, 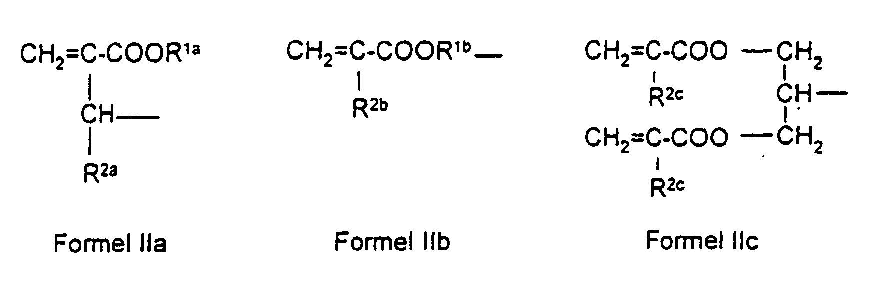 wobei die in den Formeln (IIa); (IIb) und (IIc) vorhandenen Reste unabhängig voneinander vorzugsweise eine der folgenden Bedeutungen aufweisen:
- R^{1a}:: Wasserstoff oder ein geradkettiger C₁- bis C₁₂-Alkylrest, insbesondere C₁- bis C₄- Alkylrest, ein C6- bis C₁₂-Arylrest, insbesondere C₆-Arylrest, oder C₇- bis C₁₆-AlkylarylRest, insbesondere C₇- bis C₉-Alkylarylrest;
- R^{2a}:: Wasserstoff, ein C₁- bis C₅-Alkylrest oder ein Phenylrest;
- R^{1b}:: ein C₁- bis C₁₄-Alkylenrest ist, der durch Sauerstoffatome unterbrochen sein kann, oder ein Phenylenrest;
- R^{2b}: Wasserstoff oder ein Methylrest;
- R^{2c}: Wasserstoff oder ein Methylrest.

Die Variable n ist im Fall der Formeln (IIa) und (IIb) 1, im Fall der Formel (IIc) 2.

Die Reste R sind vorzugsweise aus der Gruppe ausgewählt, die aus dem Hexamethylen-, 2,2,4-Trimethylhexamethylen-, 2,4,4-Trimethylhexamethylen-, m-Toluylen-, m-Xylylen- und Isophoronrest besteht:

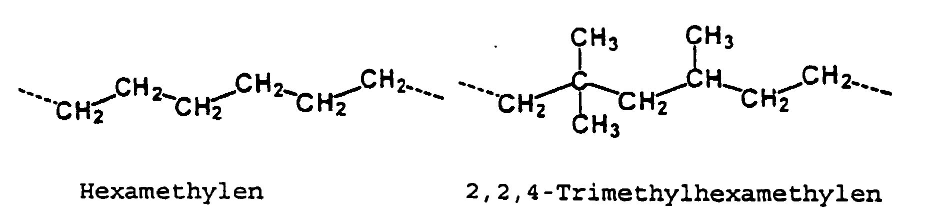

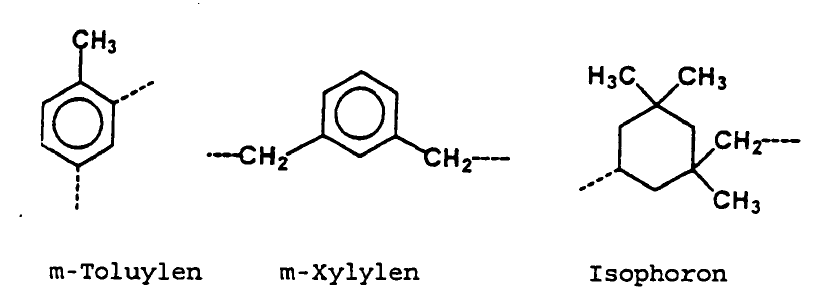

Besonders bevorzugt sind Iminooxadiazindion-Derivate gemäß Formel (I) in der der (Meth)acrylsäurerest ein Rest gemäß der Formel IIa, IIb und/oder IIc ist und mindestens einer der übrigen Reste die folgende Bedeutung hat
- R =: Hexamethylen, 2,2,4-Trimethylhexamethylen, 2,4,4-Trimethylhexamethylen oder m-Toluylen-;
- R^{1a} =: Methyl, Ethyl, Benzyl oder Phenyl;
- R^{2a} =: Wasserstoff oder Methyl;
- R^{1b} =: Ethylen, Methylethylen oder Propylen;
- R^{2b} =: Wasserstoff oder Methyl;
- R^{2c} =: Wasserstoff oder Methyl.

Ganz besonders bevorzugt sind naturgemäß solche Iminooxadiazindion-Derivate in denen alle Reste eine der genannten bevorzugten und insbesondere ein der genannten besonders bevorzugten Bedeutungen haben.

Die Iminooxadiazindion-Derivate verfügen je nach Wahl der Gruppen X, Y und Z über 3 bis 6 radikalisch polymerisierbare (Meth)acrylatgruppen, wobei über die Anzahl der polymerisierbaren Gruppen eine Steuerung der Vernetzungsdichte des gehärteten Materials möglich ist. X, Y und Z haben vorzugsweise die gleiche Bedeutung. Ebenso sind Verbindungen bevorzugt, in denen die Reste R die gleiche Bedeutung haben.

Die Iminooxadiazindion-Derivate der Formel (I) lassen sich durch Umsetzung von Diisocyanat-Trimeren mit Iminooxadiazindion-Struktur, nachfolgend auch als asymmetrische Trimere (AST) bezeichnet, mit Hydroxy(meth) acrylaten (X-OH, Y-OH bzw. Z-OH) bzw. Mischungen verschiedener Hydroxy(meth)acrylate herstellen:

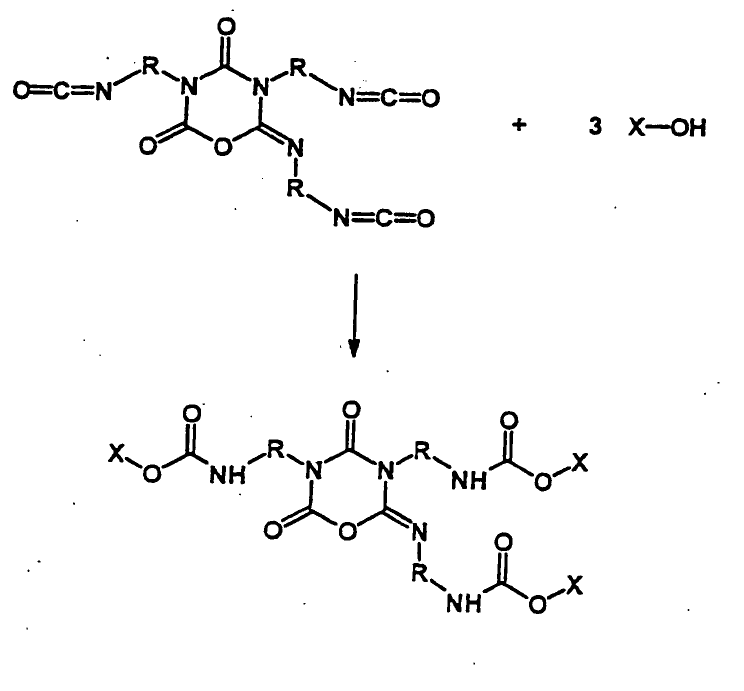

Die asymmetrischen Diisocyanat-Trimere sind analog zu den in der EP 0 798 299 A1 beschriebenen Verfahren durch Trimerisierung von käuflichen Diisocyanaten, wie Hexamethylendiisocyanat (HDI), 2,2,4- oder 2,4,4-Trimethylhexamethylendiisocyanat (TMDI), Toluylendiisocyanat (TDI), m-Xylylendiisocyanat (mXDI) oder Isophorondiisocyanat (IPDI) zugänglich. Hierbei werden Mischungen der asymmetrischen Diisocyanat-Trimere mit den entsprechenden symmetrischen Trimeren (ST) mit Isocyanurat-Struktur und Diisocyanat-Oligomeren erhalten. Unter höheren Oligomeren werden Verbindungen verstanden, die aus vier und mehr Diisocyanatmonomeren gebildet werden.

Bei den (meth)acrylatsubstituierten Iminooxadiazindion-Derivaten ist jeweils eine Isocyanatgruppe der Diisocyanate in den Iminooxadiazindionring eingebunden, die andere Isocyanatgruppe bildet mit der Hydroxylgruppe der Hydroxy(meth)acrylate jeweils eine Urethangruppe über die die (Meth) acrylatreste an den Iminooxadiazindionring gebunden sind.

Da die bei der Herstellung der Iminooxadiazindion-Derivate anfallenden symmetrischen Trimere und höheren Oligomere ebenfalls reaktive Isocyanatgruppen aufweisen, lassen sich diese auch auf die beschriebene Weise zu den entsprechenden (meth)acrylsubstituierten Derivaten umsetzen und müssen daher nicht notwendigerweise von den Iminooxadiazindion-Derivaten abgetrennt werden. Die Gesamtmenge der Derivate von symmetrischen Trimeren und höheren Oligomeren sollte jedoch möglichst gering sein und vorzugsweise maximal 50 Gew.-%, besonders bevorzugt maximal 20 Gew.-%, und ganz besonders bevorzugt maximal 5 Gew.-% betragen, jeweils bezogen auf die Gesamtmasse an Diisocyanat-Trimeren und Diisocyanat-Oligomeren. Idealerweise enthalten die Iminooxadiazindion-Derivate keine symmetrischen Trimere oder höheren Oligomere.

Die benötigten OH-funktionalisierten (Meth)acrylate sind entweder kommerziell verfügbar, wie z.B. HEMA, Hydroxypropylmethacrylat (HPMA), 2-Hydroxyethylacrylat oder GDMA, oder lassen sich auf den folgenden Wegen hergestellten (vgl. z.B. C. Ferri, Reaktionen der organischen Synthese, G. Thieme Verlag, Stuttgart 1978):

Baylis-Hillman-Reaktion von Acrylaten mit Aldehyden, die durch tertiäre Amine katalysiert wird: 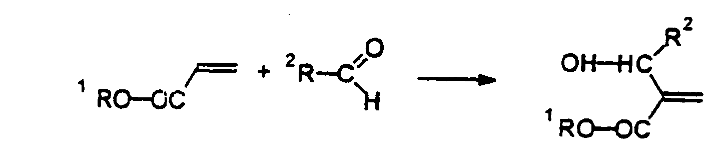 Konkretes Beispiel:

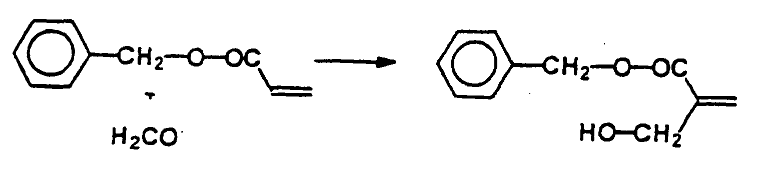

Unstöchiometrische Veresterung von Dihydroxyverbindungen mit (Meth) acrylsäure oder (Meth) acrylsäurechlorid: 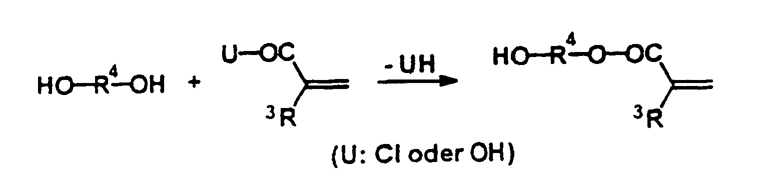 Konkretes Beispiel:

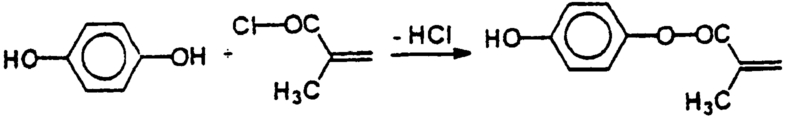

Besonders bevorzugte Beispiele für die , (meth)acrylatsubstituierten, asymmetrischen Diisocyanat-Trimere der Formel (I) sind:

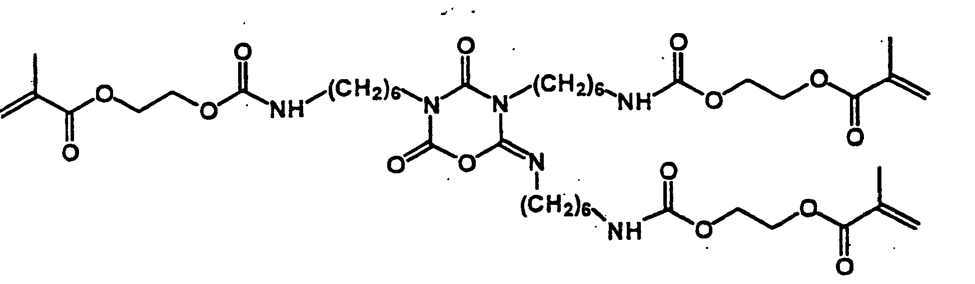

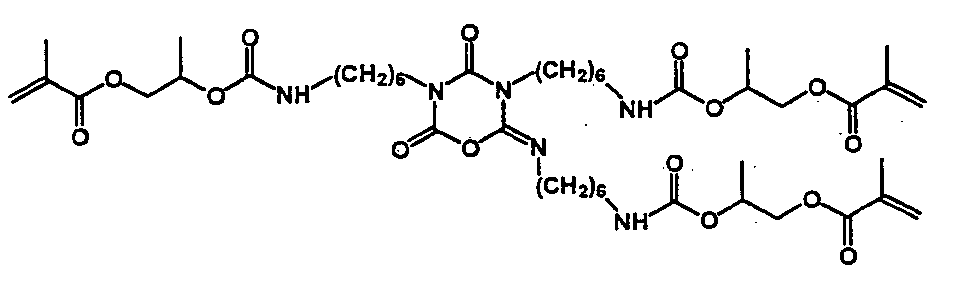

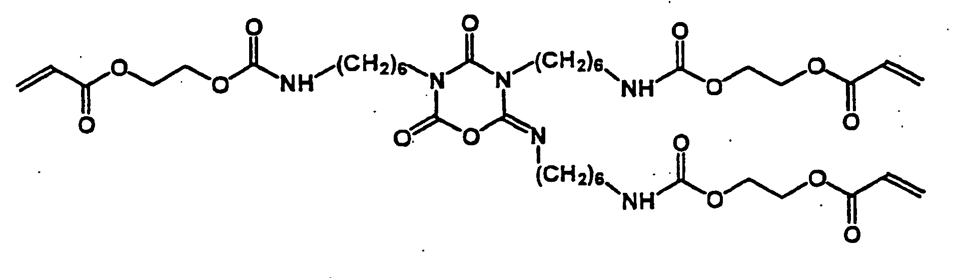

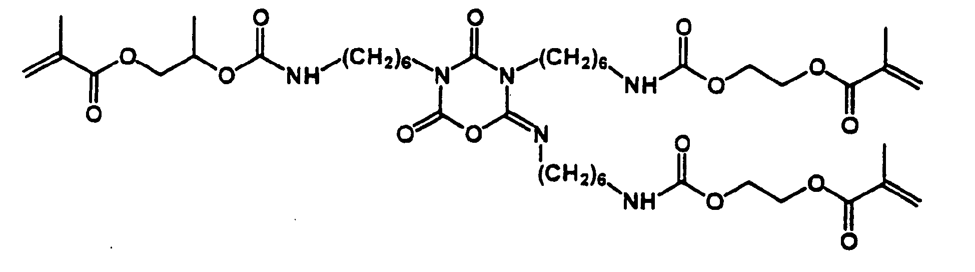

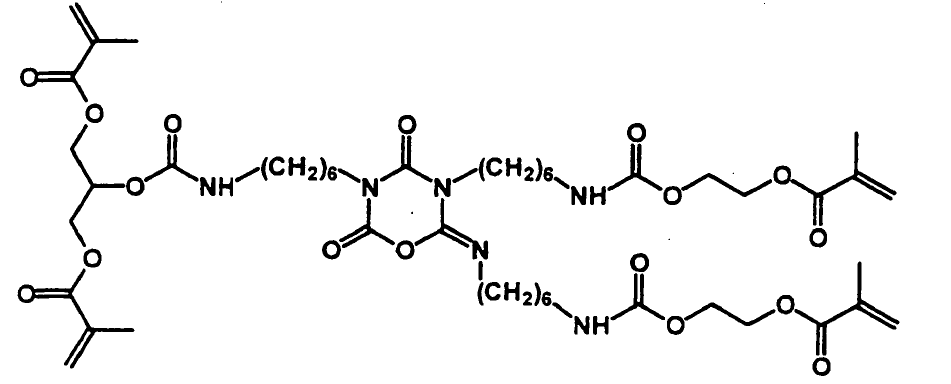

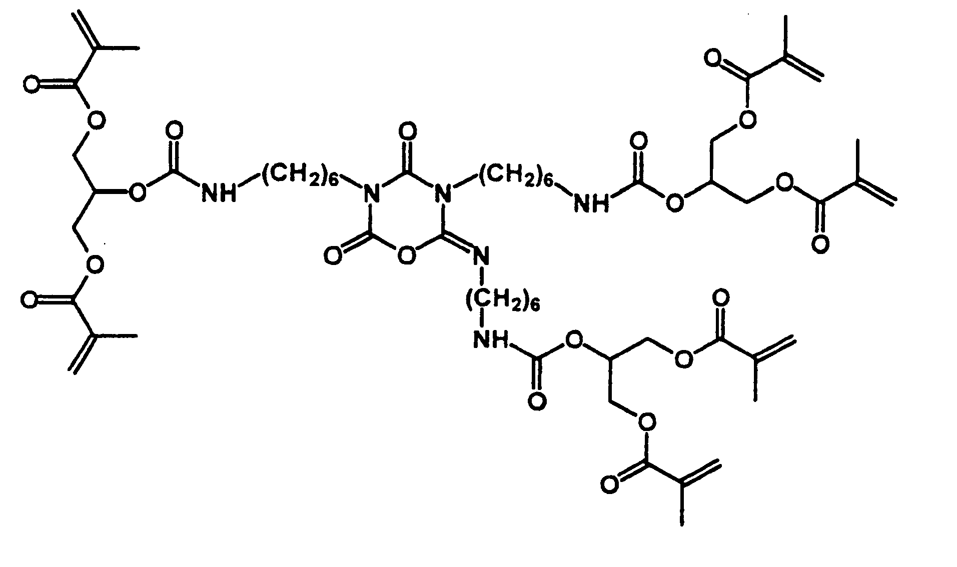

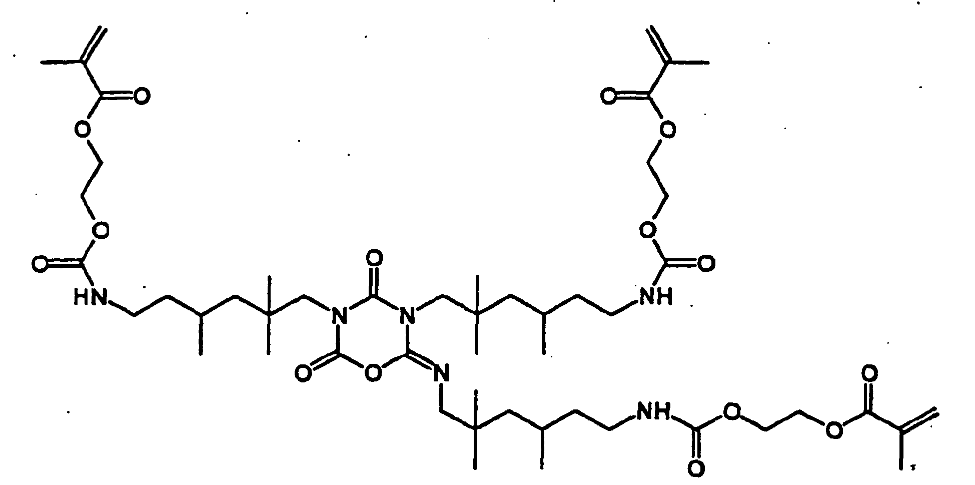

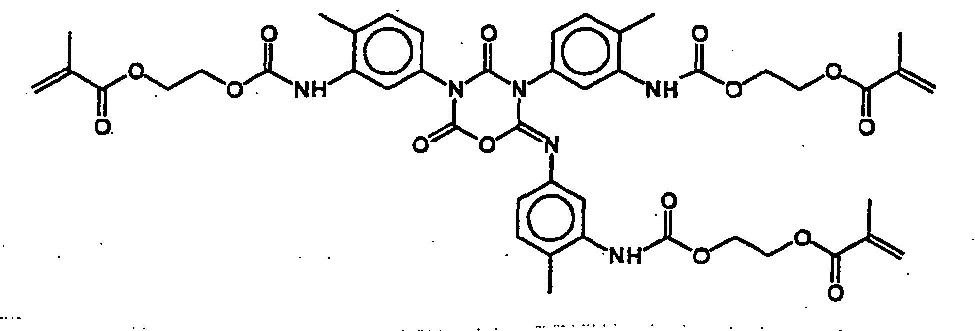

Die Iminooxadiazindion-Derivate eignen sich zur Herstellung von Polymeren, Adhäsiven und insbesondere von Dentalmaterialien, wie Füllungskompositen, Adhäsiven und Zementen, z.B. Befestigungszementen. Dabei ist vorteilhaft, daß sich auf Grund der Funktionalität der Iminooxadiazindion-Derivate eine hohe Vernetzungsdichte erreichen läßt und daß auf Grund der geringen Viskosität entweder Materialien mit einem hohen Füllstoffgehalt oder aber Materialien mit geringer Viskosität zugänglich werden. Damit lassen sich besonders gut fließbare und damit gut verarbeitbare Komposite, sogenannte "flowable" Füllungskomposite herstellen. Darüber hinaus ist die geringere Viskosität vor allem auch bei Adhäsiven vorteilhaft.

Die erfindungsgemäßen Dentalmalmaterialen enthalten mindestens ein Iminooxadiazindion-Derivat gemäß Formel (I) enthalten, in Kombination mit einem Initiator für die radikalische Polymerisation und Füllstoff. Gemäß einer besonders bevorzugten Ausführungsform enthalten die Dentalmaterialien darüber hinaus zusätzlich ein oder mehrere radikalisch polymerisierbare Monomere.

Die Iminooxadiazindion-Derivate lassen sich mit den bekannten radikalischen Initiatoren (vgl. Encylopedia of Polymer Science and Engineering, Vol. 13, Wiley-Intersci. Pub., New York etc. 1988, 754ff.) polymerisieren. Bevorzugt sind Azoverbindungen, wie Azobis(isobutyronitril) (AIBN) oder Azobis-(4-cyanvaleriansäure) oder Peroxide, wie Dibenzoylperoxid, Dilauroylperoxid, tert.-Butylperoctoat, tert.-Butylperbenzoat oder Di-(tert.-butyl)-peroxid. Als Initiatoren für die Heißhärtung eignen sich auch Benzpinakol und 2,2'-Dialkylbenzpinakole.

Weiter bevorzugt sind Photoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich, wie Benzoinether, Dialkylbenzilketale, Dialkoxyacetophenone, Acylphosphinoxide, α-Diketone wie 9,10-Phenanthrenchinon, Diacetyl, Anisil, 4,4'-Dichlorbenzil und 4,4'-Dialkoxybenzil und campherchinon.

Die Iminooxadiazindion-Derivate lassen sich allein oder in Mischung mit herkömmlichen radikalisch polymerisierbaren Monomeren, insbesondere mit difunktionellen Vernetzermonomeren polymerisieren. Für die Herstellung von Adhäsiven oder Dentalmaterialien eignen sich vor allem vernetzende bi-, oder mehrfunktionelle Acrylate bzw. Methacrylate wie z.B. UDMA, das durch Umsetzung von 1 mol 2,2,4-Trimethylhexamethylendiisocyanat mit 2 mol 2-Hydroxyethylmethacrylat (HEMA) zugänglich ist,

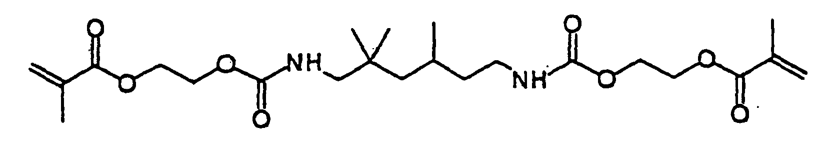

Di- oder Triethylenglycoldi(meth)acrylat (TEGDMA), Decandiol-di(meth)acrylat, Trimethylolpropantri(meth)acrylat, Pentaerythrittetra(meth)acrylat, sowie Butandioldi(meth)acrylat, 1,10-Decandioldi(meth)acrylat, 1,12-Dodecandioldi(meth)acrylat, und insbesondere 2,2-Bis-[4-(2'-hydroxy-3'-methacryloxypropoxy)phenylen]propan (Bis-GMA), die durch Veresterung von (Meth)acrylsäure mit den entsprechenden Di- oder Polyolen erhältlich sind.

Als monofunktionelle Monomere eignen sich besonders Methyl-, Ethyl-, Benzyl- und Furfurylmethacrylat sowie HEMA und Hydroxypropylmethacrylat.

Darüber hinaus können die Iminooxadiazindion-Derivate oder deren Mischungen mit anderen radikalisch polymerisierbaren Monomeren zur Verbesserung der mechanischen Eigenschaften mit organischen oder anorganischen Partikeln oder Fasern gefüllt werden. Bevorzugte anorganische partikuläre Füllstoffe sind amorphe kugelförmige Materialien auf der Basis von Mischoxiden aus SiO₂, ZrO₂ und/oder TiO₂, mikrofeine Füllstoffe, wie pyrogene Kieselsäure oder Fällungskieselsäure, die vorzugsweise eine mittlere Primärpartikelgröße zwischen 10 und 500 nm und eine BET-Oberfläche zwischen 50 und 400 m²/g aufweisen, sowie Makro- oder Minifüllstoffe, wie Quarz-, Glaskeramik- oder Glaspulver sowie röntgenopake Füllstoffe, wie Ytterbiumtrifluorid. Als faserförmige Füllstoffe sind Glasfasern, Kunstoffasern, insbesondere Polyamidfasern, und Kohlenstoffasern bevorzugt. Unter Makrofüllstoffen werden Füllstoffe mit einer mittleren Partikelgröße von 5 bis 50 µm und einer BET-Oberfläche von vorzugsweise 1 bis 10 m²/g verstanden, unter Minifüllstoffen Füllstoffe mit einer mittleren Partikelgröße von 0,5 bis 5 µm und einer BET-Oberfläche von vorzugsweise 10 bis 50 m²/g.

Weiterhin können im Bedarfsfall weitere Komponenten zugesetzt werden, vor allem Lösungsmittel, wie Wasser, Ethylacetat oder Ethanol, sowie Additive, wie Stabilisatoren, UV-Absorber, Farbstoffe oder Pigmente, sowie Gleitmittel.

Die erfindungsgemäßen Dentalmaterialien zur Verwendung als Zement enthalten:
1 bis 50 Gew.-%, besonders bevorzugt 20 bis 44 Gew.-% Iminooxadiazindion-Derivat gemäß Formel (I);
0,01 bis 5 Gew.-%, besonders bevorzugt 0,1 bis 2,0 Gew.-% Initiator für die radikalische Polymerisation;
0 bis 60 Gew.-%, besonders bevorzugt 0 bis 24 Gew.-% weiteres radikalisch polymerisierbares Monomer;
20 bis 60 Gew.-%, besonders bevorzugt 30 bis 60 Gew.-% Füllstoff.

Die erfindungsgemäßen Dentalmaterialien zur Verwendung als Füllungskomposit enthalten :
1 bis 45 Gew.-%, besonders bevorzugt 10 bis 33 Gew.-% Iminooxadiazindion-Derivat gemäß Formel (I);
0,01 bis 5 Gew.-%, besonders bevorzugt 0,1 bis 2,0 Gew.-% Initiator für die radikalische Polymerisation;
0 bis 50 Gew.-%, besonders bevorzugt 5 bis 25 Gew.-% weiteres radikalisch polymerisierbares Monomer;
30 bis 85 Gew.-%, besonders bevorzugt 40 bis 80 Gew.-% Füllstoff.

Die erfindungsgemäßen Dentalmaterialien zeichnen sich gegenüber bekannten Werkstoffen gleicher Zusammensetzung durch eine deutlich geringere Viskosität und eine bessere Fließfähigkeit aus, so daß sich die Massen sehr viel besser verarbeiten lassen. Zudem wird bei der Polymerisation eine höhere Vernetzungsdichte und eine bessere Einbindung des Iminooxadiazindion-Derivats in das Polymernetzwerk erzielt, so daß die Materialien nach der Härtung in ihren mechanischen Eigenschaften bekannten Werkstoffen überlegen sind.

Die bevorzugten Dentalwerkstoffe enthalten häufig einen mehr oder weniger hohen Füllstoffanteil, der homogen in das Material eingearbeitet werden muß, um möglichst gleichförmige Eigenschaften zu erzielen. Die erfindungsgemäßen Iminooxadiazindion-Derivate weisen eine deutlich geringere Viskosität als beispielsweise die symmetrischen Diisocyanat-Trimere auf und erleichtern so das homogene Einarbeiten von Füllstoffen ganz erheblich. Zudem gestatten sie die Verwendung größerer Füllstoffmengen, so daß eine bessere Anpassung der Eigenschaften des Werkstoffs an den gewünschten Einsatzzweck möglich ist und weitere Verbesserungen der mechanischen Eigenschaften der gehärteten Materialien ermöglicht werden.

Im folgenden wird die Erfindung anhand von Ausführungsbeispielen näher erläutert.

### Beispiele

### Beispiel 1:

### Umsetzung des asymmetrischen HDI-Triisocyanates mit HEMA

50,5 g (0,1 mol) des asymmetrischen HDI-Triisocyanates (HDI-AST) (Scherviskosität bei 40 °C = 1,3 Pas) wurden bei Raumtemperatur zu einer Lösung von 39 g (0,3 mol) HEMA, 12 mg TEMPO (2,2,6,6-Tetramethyl-piperidin-1-oxyl, Inhibitor), 25 mg MEHQ (Hydrochinonmonomethylether, Stabilisator) und 0,2 g Metatin 812 (Dibutylzinndioctoat, Initiator) in 100 ml Methylenchlorid getropft. Nach 20 h Rühren war im IR-Spektrum keine Isocyanatbande mehr zu erkennen. Das klare Reaktionsgemisch wurde 2 mal mit je 100 ml 1,0 N NaOH und 3 mal mit je 100 ml gesättigter Kochsalzlösung gewaschen. Dann wurde die organische Phase mit Natriumsulfat getrocknet, mit 20 mg MEHQ stabilisiert, und das Lösungsmittel am Rotationsverdampfer unter Lufteinleiten vollständig entfernt. Es ergaben sich 60 g (Ausbeute: 67 %) eines Produktes mit einer Scherviskosität (40 °C) von 19,7 Pas, das das Trimethacrylat HDI-AST-HEMA folgender Struktur enthält: 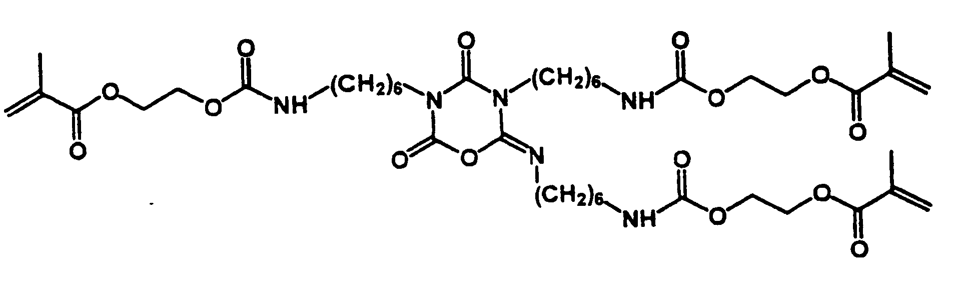 IR (ATR): 3368 (w), 2930 (m), 2858 (w), 1785 (w), 1682 (s), 1522 (m), 1458 (s) and 1163 (s) cm⁻¹.
¹H-NMR (CDCl₃) : 1,35 (br, 12 H, CH₂), 1,51 (br, 6H, CH₂), 1,63 (br, 6H, CH₂), 1,94 (s, 9H, CH₃), 3,17 und 3,34 (t, ges. 6H, CH₂N), 3,84 (t, 6H, CH₂N), 4,31 (br, 12H, CH₂O), 5,02-5,03 (br, 3H, NH), 5,59 und 6,13 (2s, je 3H, =CH₂) ppm.

### Beispiel 2 (Vergleichsbeispiel):

### Umsetzung des symmetrischen HDI-Triisocyanates mit HEMA

Analog zu Beispiel 1 wurden 50,5 g (0,1 mol) des symmetrischen HDI-Triisocyanates (HDI-ST) Desmodur N 3300 der Bayer AG (Scherviskosität bei 40 °C = 4,3 Pas) mit 39 g (0,3 mol) HEMA umgesetzt und aufgearbeitet. Es ergaben sich 69 g (Ausbeute: 77 %) eines Produktes mit einer Scherviskosität (40 °C) von 76,4 Pas, das das Trimethacrylat HDI-ST-HEMA folgender Struktur enthält: 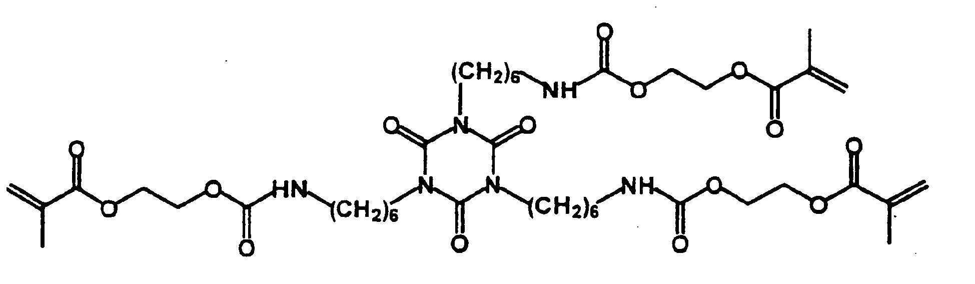 IR (ATR): 3367 (w), 2930 (m), 2858 (w), 1712 (w), 1677 (s), 1522 (m), 1459 (s) and 1164 (s) cm⁻¹.
¹H-NMR (CDCl₃) : 1,35 (br, 12 H, CH₂), 1,50 (br, 6H, CH₂), 1,63 (br, 6H, CH₂), 1,95 (s, 9H, CH₃), 3,17 (t, 6H, CH₂N), 3,75 (t, 6H, CH₂N) , 4,31 (br, 12H, CH₂O), 4,98 (br, 3H, NH), 5,59 und 6,33 (2s, je 3H, =CH₂) ppm.

### Beispiel 3:

### Umsetzung des asymmetrischen HDI-Triisocyanates mit HPMA

Analog zu Beispiel 1 wurden 50,5 g (0,1 mol) HDI-AST mit 43,3 g (0,3 mol) HPMA umgesetzt und aufgearbeitet. Es ergaben sich 56 g (Ausbeute: 59 %) eines Produktes mit einer Scherviskosität (40 °C) von 41,8 Pas, das das Trimethacrylat HDI-AST-HPMA folgender Struktur enthält: 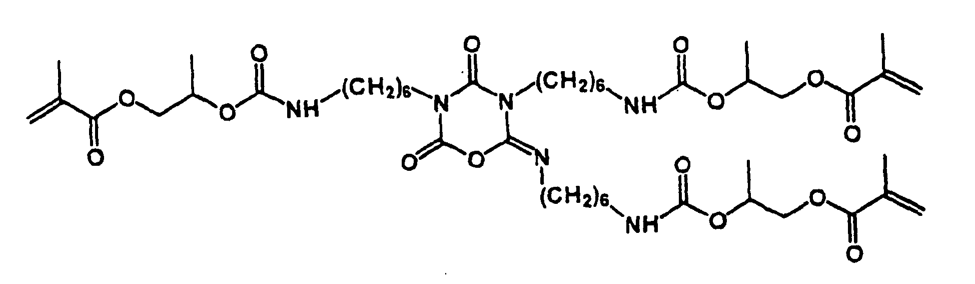 IR (ATR) : 3366 (w), 2932 (m), 2858 (w), 1785 (w), 1678 (s), 1522 (m), 1459 (s) and 1156 (s) cm⁻¹.
¹H-NMR (CDCl₃): 1,24 (br, 9H, CH₃), 1,26 (br, 12 H, CH₂), 1,51 (br, 6H, CH₂), 1,64 (br, 6H, CH₂), 1,94 (s, 9H, CH₃), 3,14 und 3,34 (t, ges. 6H, CH₂N), 3,84 (t, 6H, CH₂N), 4,14 (m, 6H, CH₂O), 4,93 (br, 3H, NH), 5,07-5,11 (m, 3H, CHO), 5,58 und 6,12 (2s, je 3H, =CH₂) ppm.

### Beispiel 4 (Vergleichsbeispiel) :

### Umsetzung des symmetrischen HDI-Triisocyanates mit HPMA

Analog zu Beispiel 1 wurden 50,5 g (0,1 mol) HDI-ST Desmodur N 3300 mit 43,3 g (0,3 mol) HPMA umgesetzt und aufgearbeitet. Es ergaben sich 64 g (Ausbeute: 67 %) eines Produktes mit einer Scherviskosität (40 °C) von 77,0 Pas, das das Trimethacrylat HDI-ST-HPMAfolgenderStruktur enthält: das Trimethacrylat 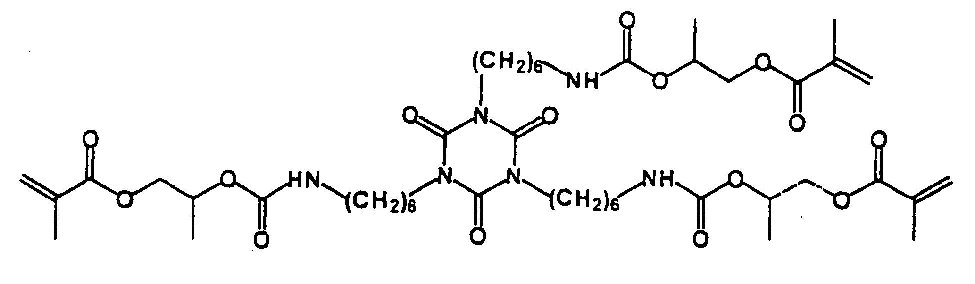 IR (ATR): 3366 (w), 2932 (m), 2858 (w), 1785 (w), 1678 (s), 1636 (w), 1522 (m), 1459 (s) and 1156 (s) cm⁻¹.
¹H-NMR (CDCl₃): 1,24 (br, 9H, CH₃), 1,27 (br, 12 H, CH₂), 1,51 (br, 6H, CH₂), 1,64 (br, 6H, CH₂), 1,94 (s, 9H, CH₃), 3,14 (t, 6H, CH₂N), 3,86 (t, 6H, CH₂N), 4,09-4,20 (m, 6H, CH₂O), 4,93 (br, 3H, NH), 5,07-5,14 (br, m, 6H, CHO und NH), 5,56 und 6,11 (2s, je 3H, =CH₂) ppm.

### Beispiel 5:

### Umsetzung des asymmetrischen HDI-Triisocyanates mit GDMA

Analog zu Beispiel 1 wurden 50,5 g (0,1 mol) HDI-AST mit 68,2 g (0,3 mol) GDMA umgesetzt und aufgearbeitet. Es ergaben sich 52 g (Ausbeute: 44%) eines Produktes mit einer Scherviskosität (40 °C) von 92,8 Pas, das das Hexamethacrylat HDI-AST-GDMA folgender Struktur enthält: 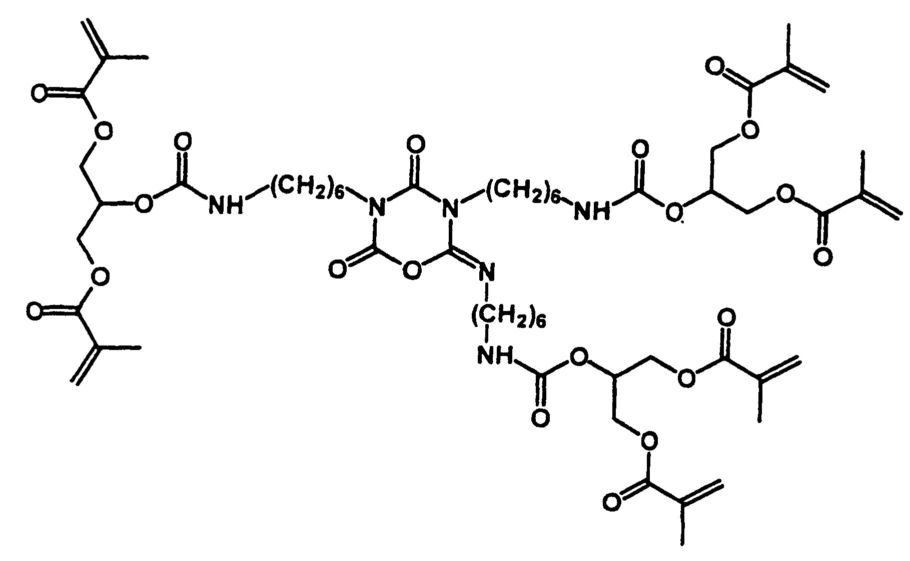 IR (ATR): 3370 (w), 2930 (m), 2858 (w), 1713 (s), 1683 (s), 1523 (m), 1460 (s) and 1149 (s) cm⁻¹.
¹H-NMR (CDCl₃) : 1,35 (br, 12 H, CH₂), 1,50 (br, 6H, CH₂), 1,63 (br, 6H, CH₂), 1,94 (s, 9H, CH₃), 3,17 und 3,34 (t, ges. 6H, CH₂N), 3,85 (t, 6H, CH₂N), 3,25-4,40 (m, 12H, CH₂O), 4,91 (br, 3H, NH), 5,27-5,31 (br, 3H, CHO), 5,60 und 6,11 (2s, je 6H, =CH₂) ppm.

### Beispiel 6 (Vergleichsbeispiel):

### Umsetzung des symmetrischen HDI-Triisocyanates mit GDMA

Analog zu Beispiel 1 wurden 50,5 g (0,1 mol) HDI-ST Desmodur N 3300 mit 68,2 g (0,3 mol) GDMA umgesetzt und aufgearbeitet. Es ergaben sich 80 g (Ausbeute: 68 %) eines Produktes mit einer Scherviskosität (40 °C) von 149,0 Pas, das das Hexamethacrylat HDI-ST-GDMA folgender Struktur enthält:

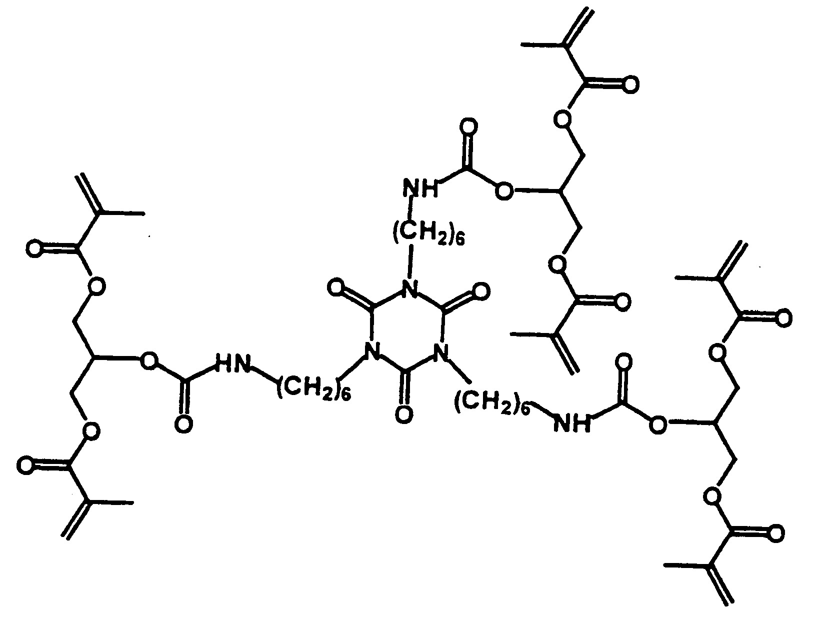

Ein Vergleich der Beispiele 1 und 2, 3 und 4 sowie 5 und 6 zeigt, daß die erfindungsgemäßen methacrylatsubstituierten asymmetrischen Diisocyanat-Trimere im Vergleich zu den entsprechenden symmetrischen Diisocyanat-Trimeren eine deutlich geringere Viskosität aufweisen.

### Beispiel 7:

### Herstellung von Kompositpasten auf der Basis der methacrylatsubstituierten Diisocyanat-Trimere der Beispiele 1 bis 4

Zur Herstellung von Kompositpasten wurden die in der nachfolgenden Tabelle angegebenen Komponenten mit Hilfe eines Dreiwalzenstuhls vom Typ Exakt (Firma Exakt Apparatebau) in den angegebenen Mengen miteinander gemischt und anschließend die komplexe Viskosität der Massen gemessen.

| Zusammensetzung der untersuchten Kompositpasten | | | | |
|---|---|---|---|---|
| Komponente | Paste 1 | Paste 2 | Paste 3 | Paste 4 |
| Trimer aus Bsp. 1 | 20 | - | - | - |
| Trimer aus Bsp. 2*) | - | 20 | - | - |
| Trimer aus Bsp. 3 | - | - | 20 | - |
| Trimer aus Bsp. 4*) | - | - | - | 20 |
| Bis-GMA | 20 | 20 | 20 | 20 |
| silanisierter Glasfüller¹⁾ | 37,8 | 37,8 | 37,8 | 37,8 |
| Sphärosil²⁾ | 10,5 | 10,5 | 10,5 | 10,5 |
| pyrogene Kieselsäure³⁾ | 0,7 | 0,7 | 0,7 | 0,7 |
| YbF₃⁴⁾ | 11,0 | 11,0 | 11,0 | 11,0 |
| Komplexe Viskosität⁵⁾ | 2,21 | 3,36 | 2,20 | 2,79 |

| | | | | |
|---|---|---|---|---|
| *) Vergleichsbeispiel | | | | |
| ¹⁾ Barium-Aluminium-Borsilikat-Glaspulver (55 Gew.-% SiO₂, 25 Gew.-% BaO, 10 Gew.-% B₂O₃, 10 Gew.-% Al₂O₃, silanisiert mit 3-Methacryloyloxypropyltrimethoxysilan, mittlere Partikelgröße ca. 1 µm (Fa. Schott) | | | | |
| ²⁾ SiO₂-ZrO₂ -Mischoxid (ca. 25 Gew.-% ZrO₂) mit einer | | | | |
| ³⁾ OX-50, Primärpartikelgröße 40 nm (Fa. Degussa) Primärpartikelgröße von 130-230 nm (Tokoyama Soda) | | | | |
| ⁴⁾ Ytterbiumfluorid (Fa. Rhone-Poulenc) | | | | |
| ⁵⁾ Bestimmt mit einem Rheometer vom Typ CVO 120 (Fa. Bohlin Instruments) bei 23 °C mit einer Scherrate von 1 Hz | | | | |

Die in der Tabelle zusammengefaßten Ergebnisse belegen, daß die erfindungsgemäßen methacrylatsubstituierten asymmetrischen Diisocyanat-Trimere im Vergleich zu den entsprechenden symmetrischen Trimeren bei gleichem Füllstoffgehalt Kompositpasten mit einer deutlich geringeren Viskosität ergeben, die eine signifikant bessere Fließfähigkeit und Verarbeitbarkeit aufweisen. Die Massen zeichnen sich durch eine gute Anpassung an die Zahnhartsubstanz aus.

Alternativ lassen sich Massen herstellen, die bei gleicher Viskosität einen höheren Füllstoffgehalt und damit bessere mechanische Eigenschaften und einen geringeren Polymerisationsschrumpf aufweisen.

## Patentansprüche

1. Dentalmaterial, das ein Iminooxadiazindion-Derivat mit der Formel (I) enthält 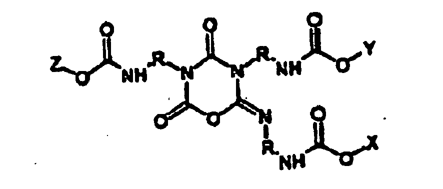 in der
X, Y und Z einen (Meth)acrylsäurerest der Formel (II) 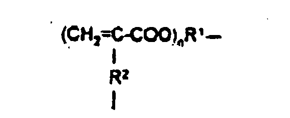 bedeuten, in der
R¹ Wasserstoff, ein aliphatischer Kohlenwasserstoffrest mit 1 bis 14 Kohlenstoffatomen, der durch Sauerstoffatome unterbrochen sein kann, oder ein aromatischer Kohlenwasserstoffrest mit 6 bis 16 Kohlenstoffatomen ist;
R² Wasserstoff, ein aliphatischer Kohlenwasserstoffrest mit 1 bis 5 Kohlenstoffatomen oder ein aromatischer Kohlenwasserstoffrest mit 6 Kohlenstoffatomen ist, wobei nur einer der Reste R¹ und R² die Bedeutung Wasserstoff haben kann;
n 1 oder 2 ist;
wobei der (Meth)acrylsäurerest der Formel (II) über R¹ oder R² an den Rest der Formel (I) gebunden und die an R² oder R¹ verbleibende Bindungsstelle durch Wasserstoff abgesättigt ist und wobei X, Y und Z gleich oder verschieden sein können;
die einzelnen Reste R gleich oder verschieden sein können und ausgewählt sind aus alicyclischen oder aromatischen Kohlenwasserstoffresten mit 5 bis 10 Kohlenstoffatomen oder aliphatischen Kohlenwasserstoffresten mit 1 bis 10 Kohlenstoffatomen, **dadurch gekennzeichnet, daß** es
| | |
|---|---|
| 1 - 50 Gew.-% | Iminooxadiazindion-Derivat gemäß Formel (I); |
| 0,01 - 5 Gew.-% | Initiator für die radikalische Polymerisation; |
| 0 - 60 Gew.-% | weiteres radikalisch polymerisierbares Monomer; |
| 20 - 60 Gew.-% | Füllstoff; oder |
| 1 - 45 Gew.-% | Iminooxadiazindion-Derivat gemäß Formel (I): |
| 0,01 - 5 Gew.-% | Initiator für die radikalische Polymerisation; |
| 0 - 50 Gew.-% | weiteres radikalisch polymerisierbares Monomer; |
| 30 - 85 Gew.-% | Füllstoff |
enthält.

2. Dentalmaterial nach Anspruch 1, **dadurch gekennzeichnet, daß** der (Meth)acrylsäurerest der Formel (II) eine Struktur der Formel (IIa), (IIb) oder (IIc) aufweist, wobei 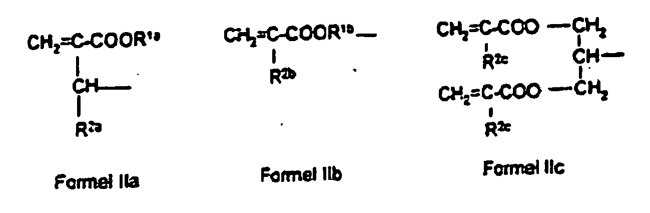
R^{1a} Wasserstoff oder ein geradkettiger C₁- bis C₁₂-Alkyl- rest, ein C₆- bis C₁₂-Arylrest oder C₇- bis C₁₆- Alkylaryl-Rest ist;
R^{2a} Wasserstoff, ein C₁- bis C₅-Alkylrest oder ein Phenyl- rest ist;
R^{1b} ein C₁- bis C₁₄-Alkylenrest ist, der durch Sauerstoff- atome unterbrochen sein kann, oder ein Phenylenrest ist;
R^{2b} Wasserstoff oder ein Methylrest ist;
R^{2c} Wasserstoff oder ein Methylrest ist.

3. Dentalmaterial nach Anspruch 1 oder 2, **dadurch gekennzeichnet, daß** die Reste R aus der Gruppe ausgewählt sind, die aus dem Hexamethylen-, 2,2,4-Trimethylhexamethylen-, 2,4,4-Trimethylhexamethylen-, m-Toluylen-, m-Xylylen- oder Isophoronrest besteht.

4. Dentalmaterial nach einem der Ansprüche 1 bis 3, **dadurch gekennzeichnet, daß** mindestens einer der Reste eine der folgenden Bedeutungen hat
R = Hexamethylen, 2,2,4-Trimethylhexamethylen, 2,4,4-Trimethylhexamethylen oder m-Toluylen-;
R^{1a} = Methyl, Ethyl, Benzyl oder Phenyl;
R^{2a} = Wasserstoff oder Methyl;
R^{1b} = Ethylen, Methylethylen oder Propylen;
R^{2b} = Wasserstoff oder Methyl;
R^{2c} = Wasserstoff oder Methyl.

5. Dentalmaterial nach einem der Ansprüche 1 bis 4, **dadurch gekennzeichnet, daß** X, Y und Z die gleiche Bedeutung haben.

6. Dentalmaterial nach einem der Ansprüche 1 bis 5, **dadurch gekennzeichnet, daß** es partikulären und/oder faserförmigen Füllstoff enthält.

7. Verwendung eines Dentalmaterials gemäß einem der Ansprüche 1 bis 6 Dentalmaterial, das
| | |
|---|---|
| 1 - 50 Gew.-% | Iminooxadiazindion-Derivat gemäß Formel (I); |
| 0,01-5 Gew.-% | Initiator für die radikalische Polymerisation; |
| 0 - 60 Gew.-% | weiteres radikalisch polymerisierbares Monomer; |
| 20 - 60 | Gew.-%Füllstoff enthält, als Zement. |

8. Verwendung eines Dentalmaterials gemäß einem der Ansprüche 1 bis 6 Dentalmaterial, das
| | |
|---|---|
| 1 - 45 Gew.-% | Iminooxadiazindion-Derivat gemäß Formel (I); |
| 0,01-5 Gew.-% | Initiator für die radikalische Polymerisation; |
| 0 - 50 Gew.-% | weiteres radikalisch polymerisierbares Monomer; |
| 30-85 Gew.-% | Füllstoff enthält, als Füllungskomposit. |

## Claims

1. Dental material comprising an iminooxadiazine dione derivative with the Formula (I) 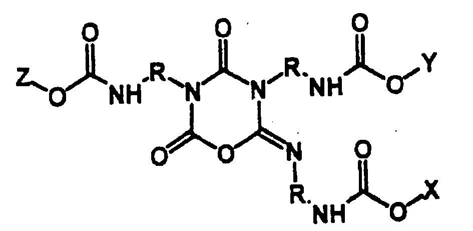 in which X, Y and Z mean a (meth)acrylic acid group of Formula (II) 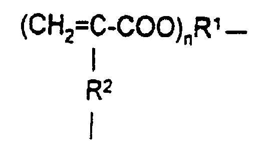 in which
R¹ is hydrogen, an aliphatic hydrocarbon group containing 1 to 14 carbon atoms which can be interrupted by oxygen atoms, or an aromatic hydrocarbon group containing 6 to 16 carbon atoms;
R² is hydrogen, an aliphatic hydrocarbon group containing 1 to 5 carbon atoms or an aromatic hydrocarbon group containing 6 carbon atoms, wherein only one of the groups R¹ and R² can have the meaning hydrogen;
n is 1 or 2;
wherein the (meth)acrylic acid group of Formula (II) is bonded through R¹ or R² to the group of Formula (I) and the remaining bonding position on R² or R¹ is saturated by hydrogen, and wherein X, Y and Z can be the same or different;
the individual groups R can be the same or different and selected from alicyclic or aromatic hydrocarbon groups containing 5 to 10 carbon atoms or aliphatic hydrocarbon groups containing 1 to 10 carbon atoms, **characterised in that** it comprises
| | |
|---|---|
| 1 - 50 wt.% | iminooxadiazine dione derivative according to Formula (I); |
| 0.01 - 5 wt.% | initiator for the radical polymerisation; |
| 0 - 60 wt.% | additional radically polymerisable monomer; |
| 20 - 60 wt.% | filler; |
or
| | |
|---|---|
| 1 - 45 wt.% | iminooxadiazine dione derivative according to Formula (I); |
| 0.01 - 5 wt.% | initiator for the radical polymerisation; |
| 0 - 50 wt.% | additional radically polymerisable monomer; |
| 30 - 60 wt.% | filler. |

2. Dental material according to claim 1, **characterised in that** the (meth)acrylic acid group of Formula (II) has a structure of Formula (IIa), (IIb) or (IIc), wherein 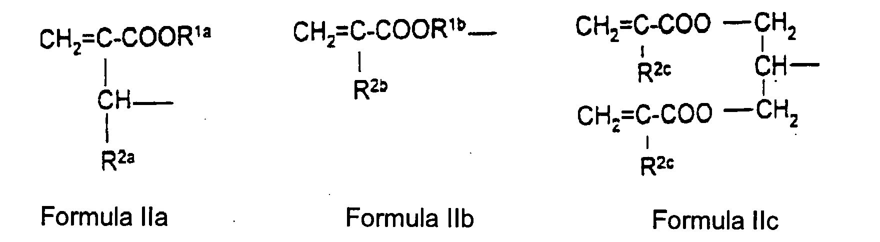
R^{1a} is hydrogen or a straight chain C₁- to C₁₂- alkyl group, a C₆- to C₁₂-aryl group or a C₇- to C₁₆- alkylaryl group;
R^{2a} is hydrogen, a C₁- to C₅- alkyl group or a phenyl group;
R^{1b} is a C₁- to C₁₄- alkylene group that can be interrupted by oxygen atoms, or is a phenylene group;
R^{2b} is hydrogen or a methylene group;
R^{2c} is hydrogen or a methyl group.

3. Dental material according to claim 1 or 2, **characterized in that** the R groups are selected from the group that consists of the hexamethylene, 2,2,4-trimethylhexamethylene, 2,4,4-trimethylhexamethylene, m-toluylene, m-xylylene or isophorone groups.

4. Dental material according to one of claims 1 to 3, **characterised in that** at least one of the groups has one of the following meanings
| | |
|---|---|
| R = | hexamethylene, 2,2,4-trimethylhexamethylene, 2,4,4-trimethylhexamethylene or m-toluylene; |
| R^{1a} = | methyl, ethyl, benzyl or phenyl; |
| R^{2a} = | hydrogen or methyl; |
| R^{1b} = | ethylene, methylethylene or propylene; |
| R^{2b} = | hydrogen or methyl; |
| R^{2c} = | hydrogen or methyl. |

5. Dental material according to one of claims 1 to 4, wherein X, Y and Z have the same meaning.

6. Dental material according to one of claims 1 to 5 comprising particulate and/or fibrous filler.

7. Use of a dental material according to one of claims 1 to 6 as a cement comprising
| | |
|---|---|
| 1 - 50 wt.% | iminooxadiazine dione derivative according to Formula (I); |
| 0.01 - 5 wt.% | initiator for the radical polymerisation; |
| 0 - 60 wt.% | additional radically polymerisable monomer; |
| 20 - 60 wt.% | filler. |

8. Use of a dental material according to one of claims 1 to 6 as a composite filling comprising
| | |
|---|---|
| 1 - 45 wt.% | iminooxadiazine dione derivative according to Formula (I); |
| 0.01 - 5 wt.% | initiator for the radical polymerisation; |
| 0 - 50 wt.% | additional radically polymerisable monomer; |
| 30 - 85 wt.% | filler. |

## Revendications

1. Matériau dentaire contenant un dérivé d'imino-oxadiazine-dione, de formule (I) : 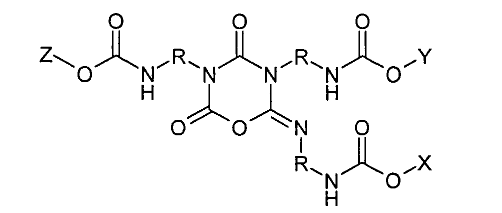 dans laquelle
X, Y et Z représentent chacun un reste de type acide acrylique ou méthacrylique, de formule (II) : 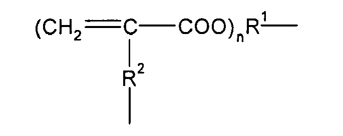 dans laquelle
- R¹ représente un atome d'hydrogène, un reste d'hydrocarbure aliphatique comportant 1 à 14 atomes de carbone, dont la chaîne peut être interrompue par un ou des atome(s) d'oxygène, ou un reste d'hydrocarbure aromatique comportant 6 à 16 atomes de carbone,
- R² représente un atome d'hydrogène, un reste d'hydrocarbure aliphatique comportant 1 à 5 atomes de carbone, ou un reste d'hydrocarbure aromatique comportant 6 atomes de carbone,
étant entendu que seul l'un des symboles R¹ et R² peut représenter un atome d'hydrogène,
- et l'indice n vaut 1 ou 2 ;
étant entendu que les restes de type acide acrylique ou méthacrylique de formule (II) sont liés au reste de la molécule de formule (I) par l'intermédiaire du reste R¹ ou du reste R² l'emplacement de liaison restant au niveau du reste R¹ ou R² étant occupé par un atome d'hydrogène, et que les restes symbolisés par X, Y et Z peuvent être identiques ou différents ;
et les symboles R représentent des entités qui peuvent être identiques ou différentes et qui sont choisies parmi les restes d'hydrocarbures alicycliques ou aromatiques comportant 5 à 10 atomes de carbone et les restes d'hydrocarbures aliphatiques comportant 1 à 10 atomes de carbone, lequel matériau dentaire est **caractérisé en ce qu'**il contient :
- 1 à 50 % en poids de dérivé d'imino-oxadiazine-dione de formule (I) ;
- 0,01 à 5 % en poids d'un amorceur de polymérisation radicalaire ;
- 0 à 60 % en poids d'un autre monomère polymérisable par voie radicalaire ;
- et 20 à 60 % en poids d'une charge ;
ou bien :
- 1 à 45 % en poids de dérivé d'imino-oxadiazine-dione de formule (I) ;
- 0,01 à 5 % en poids d'un amorceur de polymérisation radicalaire ;
- 0 à 50 % en poids d'un autre monomère polymérisable par voie radicalaire ;
- et 30 à 85 % en poids d'une charge.

2. Matériau dentaire conforme à la revendication 1, **caractérisé en ce que** le reste de type acide acrylique ou méthacrylique de formule (II) possède une structure de formule (IIa), (IIb) ou (IIc) : 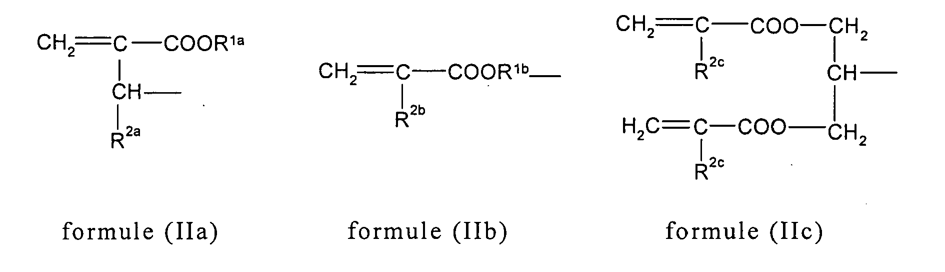 dans lesquelles formules :
- R^{1a} représente un atome d'hydrogène ou un groupe alkyle à chaîne droite en C₁₋₁₂, aryle en C₆₋₁₂ ou alkyl-aryle en C₇₋₁₆ ;
- R^{2a} représente un atome d'hydrogène, un groupe alkyle en C₁₋₅ ou un groupe phényle ;
- R^{1b} représente un groupe alcanediyle en C₁₋₁₄, dont la chaîne peut être interrompue par un ou des atome(s) d'oxygène, ou un groupe phényle ;
- R^{2b} représente un atome d'hydrogène ou un groupe méthyle ;
- et R^{2c} représente un atome d'hydrogène ou un groupe méthyle.

3. Matériau dentaire conforme à la revendication 1 ou 2, **caractérisé en ce que** les restes symbolisés par R sont choisis dans l'ensemble constitué par les restes hexaméthylène, 2,2,4-triméthyl-hexaméthylène, 2,4,4-triméthyl-hexaméthylène, m-tolylène et m-xylylène et les restes d'isophorone.

4. Matériau dentaire conforme à l'une des revendications 1 à 3, **caractérisé en ce qu'**au moins l'un des symboles a l'une des significations suivantes :
- R représente un reste hexaméthylène, 2,2,4-triméthyl-hexaméthylène, 2,4,4-triméthyl-hexaméthylène, ou m-tolylène ;
- R^{1a} représente un groupe méthyle, éthyle, benzyle ou phényle ;
- R^{2a} représente un atome d'hydrogène ou un groupe méthyle ;
- R^{1b} représente un groupe éthanediyle, méthyl-éthanediyle ou propanediyle ;
- R^{2b} représente un atome d'hydrogène ou un groupe méthyle ;
- et R^{2c} représente un atome d'hydrogène ou un groupe méthyle.

5. Matériau dentaire conforme à l'une des revendications 1 à 4, **caractérisé en ce que** les symboles X, Y et Z ont la même signification.

6. Matériau dentaire conforme à l'une des revendications 1 à 5, **caractérisé en ce qu'**il contient une charge qui se présente sous forme de particules et/ou de fibres.

7. Utilisation d'un matériau dentaire, conforme à l'une des revendications 1 à 6, qui contient :
- 1 à 50 % en poids de dérivé d'imino-oxadiazine-dione de formule (I),
- 0,01 à 5 % en poids d'un amorceur de polymérisation radicalaire,
- 0 à 60 % en poids d'un autre monomère polymérisable par voie radicalaire,
- et 20 à 60 % en poids d'une charge,
en tant que ciment.

8. Utilisation d'un matériau dentaire, conforme à l'une des revendications 1 à 6, qui contient :
- 1 à 45 % en poids de dérivé d'imino-oxadiazine-dione de formule (I),
- 0,01 à 5 % en poids d'un amorceur de polymérisation radicalaire,
- 0 à 50 % en poids d'un autre monomère polymérisable par voie radicalaire,
- et 30 à 85 % en poids d'une charge,
en tant que matériau composite de remplissage.
